**Europäisches Patentamt**

**European Patent Office**

**Office européen des brevets**

(19)

(11) Veröffentlichungsnummer: **0 232 416**
**B1**

(12) **EUROPÄISCHE PATENTSCHRIFT**

(45) Veröffentlichungstag der Patentschrift: **11.07.90**

(21) Anmeldenummer: **86905766.1**

(22) Anmeldetag: **22.08.86**

(86) Internationale Anmeldenummer:
**PCT/EP86/00494**

(87) Internationale Veröffentlichungsnummer:
**WO 87/01133 26.02.87 Gazette 87/05**

(51) Int. Cl.⁵: **C 12 Q 1/00**, C 12 Q 1/26,
C 12 Q 1/60

(54) **VERFAHREN ZUM NACHWEIS VON VERBINDUNGEN, DIE KATALYSIERT DURCH ENZYME ELEKTRONEN ABGEBEN ODER AUFNEHMEN KÖNNEN.**

(30) Priorität: **22.08.85 DE 3530076**

(43) Veröffentlichungstag der Anmeldung:
**19.08.87 Patentblatt 87/34**

(45) Bekanntmachung des Hinweises auf die Patenterteilung:
**11.07.90 Patentblatt 90/28**

(84) Benannte Vertragsstaaten:
**AT BE CH DE FR GB IT LI LU NL SE**

(56) Entgegenhaltungen:
**GB-A-2 078 369**
**US-A-4 040 908**

**Journal of Chromatography, vol. 344, 08.11.1985, (Elseviers Science Publishers, Amsterdam, NL), N.Kaneda et al.: "Highly sensitive assay for acetylcholinesterose asctivity by high-performance liquid chromatography with electrochemical detection", pp 93-100**

(73) Patentinhaber: **Max-Planck-Gesellschaft zur Förderung der Wissenschaften e.V.**
**Bunsenstrasse 10**
**D-3400 Göttingen (DE)**

(72) Erfinder: **STADLER, Herbert**
**Obere Reihe 12**
**D-3430 Niemetal (DE)**
Erfinder: **NESSELHUT, Thomas**
**Seb.-Kneipp-Str. 42 b**
**D-3430 Witzenhausen 4 (DE)**

(74) Vertreter: **Huber, Bernhard, Dipl.-Chem. et al Patentanwälte H. Weickmann, Dr. K. Fincke F.A. Weickmann, B. Huber Dr. H. Liska, Dr. J. Prechtel Möhlstrasse 22 Postfach 860 820 D-8000 München 86 (DE)**

(56) Entgegenhaltungen:
**Chemical Abstracts, vol.104, no. 5, 03.02.86, (Columbus, Ohio, US); Y. Toshio et al.: "Amperometric detection of acetylcholine & choline in a liquid chromatographic system with an immobilized enzyme reactor", see page 286, abstract 31251c**

EP 0 232 416 B1

(56) Entgegenhaltungen:

Chemical Abstracts, vol.103, no. 23, 09.12.85
(Columbus, Ohio, US); F.P: Bymaster et al
"Measurements of acetylcholine and choline in
brain by HPLC with electro-chemical detection",
see p. 304, abstract 192712y

Chemical Abstracts, vol.104, no.2, 13.01.1986
(Columbus, Ohio, US); Y. Toshio et al.: "High-
performance liquid chromatographic
determination of acetylcholine and choline by
the use of immobilized enzyme electrode as the
detector", see page 588, abstract 14277y

**Beschreibung**

Gerade im medizinischen Bereich ist es erforderlich, Verbindungen nachzuweisen, die in sehr geringen Konzentrationen im Blut, im Urin, in anderen Körperflüssigkeiten oder in Lösungen vorliegen. Oft sind geringste Mengen von Verbindungen schon ein Hinweis auf physiologische Vorgänge im Körper, aus denen der Arzt dann auf Krankheiten oder Störungen des Stoffwechsels schließen kann. Es ist deshalb notwendig, Verbindungen bis unter den Picogrammbereich nachweisen zu können. Gerade in den Körperflüssigkeiten sind aber sehr viele Verbindungen, die ähnlich aufgebaut sind, vorhanden. Es ist deshalb erforderlich, nicht nur sehr genaue Nachweismethoden, sondern auch sehr spezifische Nachweismethoden anzuwenden. Als sehr genaue und gute Trennungsmethode hat sich dabei die HPLC bewährt. Mit Hilfe von HPLC lassen sich Substanzen sehr fein und sehr schnell auftrennen.

Da es weiterhin bekannt ist, daß mit Hilfe der Enzymanalytik der spezifische Nachweis bestimmter Verbindungen möglich ist, wurde von Potter et al. in Journal of Neurochemistry, Vol. 41, Nr. 1 (1983), Seiten 188 bis 194 vorgeschlagen, Acetylcholin und Cholin nachzuweisen, indem man eine Lösung, die Acetylcholin und Cholin enthält, zuerst über eine HPLC-Säule schickt, anschließend in dem Eluat, das aus der HPLC-Säule kommt, den pH neu einstellt, Enzyme hinzugibt und nach der enzymatischen Umsetzung, die einige Minuten dauert, in der den Reaktionscoil verlassenden Lösung gebildetes $H_2O_2$ elektrochemisch nachweist. Dabei liegt die Nachweisgrenze für Cholin und Acetylcholin bei 1 bzw. 2 Picomol.

Es ist nun aber wünschenswert, die Nachweisgrenze für Substanzen wie Cholin und Acetylcholin und andere Verbindungen noch weiter herabzudrücken, ohne die Spezifität des Nachweises zu beeinflussen. Es war daher Ziel der Erfindung, ein Verfahren zu schaffen, das sehr spezifisch ist und es ermöglicht, Verbindungen noch in weit geringeren Konzentrationen, als es bisher möglich war, nachzuweisen.

Dieses Ziel wird erreicht durch ein Verfahren zum Nachweis von Verbindungen, die Substrate für eine $H_2O_2$-liefernde oxidoreduktase darstellen, unter Verwendung von HPLC und elektrochemischen Nachweismethoden, das dadurch gekennzeichnet ist, daß man eine Probe, die eine oder mehrere zu bestimmende Verbindungen enthält, die Substrate für eine $H_2O_2$-liefernde Oxidoreduktase darstellen, auf eine HPLC-Säule gibt, dann das Eluat durch eine, mit einer Trägersubstanz gepackten Minisäule leitet, an der die Oxidoreduktase und gegebenenfalls Hilfsenzyme gebunden sind, die Verbindungen in Substrate für eine $H_2O_2$-liefernde Oxidoreduktase umwandeln, wobei der pH des Eluats nicht verändert wird und anschließend die in der die Minisäule verlassende Lösung, die von der Oxidoreduktase oxidierte oder reduzierte Verbindung durch elektrochemische Detektion quantitativ bestimmt.

Mit Hilfe dieses erfindungsgemäßen Verfahrens ist es überraschenderweise möglich, Verbindungen, die Substrate für eine $H_2O_2$-liefernde Oxidoreduktase darstellen, bis zu einer Konzentration von 0,1 Picomol nachzuweisen. Das bedeutet gegenüber dem bisher bekannten Verfahren eine Verbesserung um den Faktor 10 bis 15. Dazu genügt es, sehr kleine Minisäulen mit einem Bettvolumen von nur 20 bis 150 Mikroliter zu benützen. Das Verfahren läßt sich für alle Verbindungen anwenden, die durch HPLC aufgetrennt werden können und die die Substrate für eine $H_2O_2$-liefernde Oxidoreduktase darstellen und die nach der enzymatischen Umsetzung elektrochemisch nachweisbar sind.

Die Auftrennung über HPLC erfolgt in an sich bekannter Weise. Dabei wird die Säulenpackung und das Elutionsmittel je nach den aufzutrennenden Verbindungen ausgewählt. Die entsprechenden Packungen und Elutionsmittel sind dem Fachmann auf diesem Gebiet bekannt.

Das Eluat aus der HPLC-Säule wird dann direkt in eine Minisäule, die eine Größe von etwa 2 bis 8 x 0,05 bis 0,1 cm haben kann, geleitet, die mit einer Trägersubstanz gepackt ist, an der die Oxidoreduktase und gegebenenfalls Hilfsenzyme gebunden sind, die Verbindungen in Substrate für eine $H_2O_2$- liefernde Oxidoreduktase umwandeln.. Als Trägersubstanz besonders geeignet ist dabei Agarose. Es sind jedoch andere, an sich bekannte Trägersubstanzen verwendbar. An die Trägersubstanz ist ein Enzym gebunden, das die für die nachzuweisende Verbindung gewünschte Reaktion spezifisch katalysiert. Es ist auch möglich, weitere Enzyme oder eine Kombiantion von Enzymen zu binden, die eine Reaktion katalysieren, die zu der Verbindung führt, die ein Substrat für eine $H_2O_2$-liefernde Oxidoreduktase darstellt und dann durch die immobilisierte Oxidoreduktase weiter umgesetzt wird. So können z.B. in der HPLC-Säule verschiedene Derivate einer Verbindung aufgetrennt werden. Die Verbindung und ihre nacheinander eluierten Derivate können dann über eine enzymatisch durch die Oxidoreduktase oxidierte oder reduzierte Nachweisverbindung elektrochemisch nachgewiesen werden, wenn die einzelnen Derivate von einem Enzym vorher in die Verbindung umgewandelt wurden.

In der von den Enzymen umgesetzten Lösung kann sodann die von dem Enzym oxidierte oder reduzierte Verbindung durch elektrochemische Detektion quantitativ bestimmt werden. Dazu sind die an sich bekannten elektrochemischen Verfahren geeignet. So ist es beispielsweise möglich, gebildetes $H_2O_2$ mit Platinelektroden quantitativ zu bestimmen. Ebenso läßt sich gebildetes NADH oder NADPH über elektrochemische Detektion quantitativ erfassen.

Bei einer bevorzugten Ausführungsform des erfindungsgemäßen Verfahrens werden Acetylcholin und Cholin nebeneinander nachgewiesen. Dazu wird die Lösung, die Acetylcholin und Cholin enthält, über eine HPLC-Säule gegeben. Acetylcholin und Cholin werden in der HPLC-Säule aufgetrennt und nacheinander eluiert. Das Eluat wird durch eine Minisäule geleitet, die mit Agarose gepackt ist. An der Agarose sind Acetylcholinesterase und Cholinoxidase gebunden. Das zuerst eluierte Cholin wird durch die Cholinoxidase zu $H_2O_2$ und Betain umgesetzt. Das entstehende $H_2O_2$ wird mit einem elektrochemischen Detektor, der mit Platinelektroden und einer Ag/AgCl-Referenzelektrode ausgestattet ist, nachgewiesen. Das danach eluierte Acetylcholin wird durch die Acetylcholinesterase in Essigsäure und Cholin gespalten. Das Cholin wird durch die Cholinoxidase oxidiert und das dabei gebildete $H_2O_2$ wird wiederum elektrochemisch nachgewiesen.

In einer weiteren bevorzugten Ausführungsform des erfindungsgemäßen Verfahrens werden Cholesterin und Cholesterinester nachgewiesen. Dabei werden über HPLC Cholesterin und Cholesterinester aufgetrennt. In einer Minisäule, die mit Agarose gepackt ist, woran Cholesterinesterase und Cholesterinoxidase gebunden sind, wird das Cholesterin oxidiert. Die außerdem eluierten Cholesterinester werden enzymatisch hydrolysiert und das entstehende Cholesterin wird dann oxidiert. Das bei der Oxidationsreaktion entstehende $H_2O_2$ wird dann wieder elektrochemisch detektiert.

Mit Hilfe des erfindungsgemäßen Verfahrens ist es möglich, z. B. Neurotransmitter, Leukotriene, Steroidhormone, Cholesterin und Cholesterinester und Substrate von Reduktasen nachzuweisen. Besonders geeignet sind für das erfindungsgemäße Verfahren Substanzen, die ein Substrat für eine $H_2O_2$-liefernde Oxidase darstellen.

Vorteil des erfindungsgemäßen Verfahrens ist es, daß es sehr einfach durchgeführt werden kann. Das Eluat aus der HPLC-Säule kann unverändert in die Minisäule und von dort unverändert zu der elektrochemischen Detektion geleitet werden. Eine Änderung des pH's ist überraschenderweise nicht notwendig. Bisher wurde angenommen, daß Elution und Enzymreaktion bei verschiedenen pH-Werten stattfinden müßten, da für beide Verfahren in der Regel verschiedene pH-Optima gegeben sind. Überraschenderweise stellte sich jedoch heraus, daß bei Anwendung desselben pH-Bereiches im gesamten Verfahren die Nachweisgrenze um den Faktor 10 bis 15 verbessert werden konnte, ohne daß die Spezifität dadurch verschlechtert worden wäre. Es ist also möglich, erfindungsgemäß Substanzen so spezifisch und empfindlich nachzuweisen, wie es bisher nicht möglich war.

Erfindungsgemäß wird ein Verfahren zur Verfügung gestellt, mit dem es möglich ist, sehr einfach, schnell, genau und empfindlich Verbindungen, insbesondere in Körperflüssigkeiten, nachzuweisen. Selbstverständlich eignet sich das erfindungsgemäße Verfahren auch für allgemeine analytische Verfahren, wie z. B. den Nachweis bestimmter Verbindungen in Lebensmitteln, Kunststoffen etc.

Die Erfindung soll noch durch Beispiele erläutert werden.

Beispiel 1

Gewebe aus Meerschweinchengehirn, von Torpedo und isolierte synaptische Vesikel von Torpedo wurden in kalter, 0,4 molarer $HClO_4$ homogenisiert und dann mit einmolarer KOH auf einen pH von 5,0 eingestellt und mit 10000 g/min zentrifugiert. Die Überstände (20 µl) wurden direkt verwendet.

Es wurden Standardlösungen aus Acetylcholinperchlorat und Cholinchlorid wöchentlich frisch hergestellt und bei -20°C eingefroren; bei einem pH von 5,0 aufbewahrt und direkt vor Gebrauch jeweils aufgetaut.

Für das HPLC-Gerät wurde eine Druckeinheit verwendet, wodurch ein konstanter Durchfluß durch einen Stickstoffzylinder gewährleistet war. Die Säule (15 x 0,2 cm) war gepackt mit kugelförmigem Kieselgel, das $SO_3H$-Gruppen trug. Die Teilchengröße betrug 5 µm. Es wurde eluiert mit 700 µl/min und einem konstanten Druck von 100 bar. Als Elutionsmittel wurde ein Puffer, der 50 mmol Natriumphosphat und 3 mmol Tetramethylammoniumchlorid in Wasser enthielt und einen pH von 7,6 hatte, verwendet.

Acetylcholesterinesterase (200 Einheiten) aus Electrophorus electricus und Cholinoxidase (50 U) Arthrobacter clobiformis wurden an 5 ml vorgequollene, mit CNBr aktivierte Agarose gebunden. Ein Teil der Aufschlämmung wurde dreimal mit dem Elutionspuffer gewaschen und dann in eine Glasminisäule (4 x 0,1 cm, Volumen 125 µl) mit einem keramischen Filter eingebracht, die mit dem Ausfluß der HPLC-Säule verbunden war.

Als elektrochemischer Detektor wurden eine Platinelektrode und eine Ag/AgCl-Referenzelektrode verwendet. Der Detektor wurde bei 500 mV betrieben.

Die erhaltenen Ergebnisse wurden verglichen mit Ergebnissen, die mit einem Acetylcholinbioassay unter Verwendung von Blutegelrückenmuskel erhalten wurden.

Acetylcholin und Cholin wurden zuerst durch HPLC aufgetrennt, dann über eine Minisäule, die immobilisierte Acetylcholinesterase und Cholinoxidase gebunden hatte, geleitet. Acetylcholin wurde hier zu Cholin und Essigsäure hydrolysiert. Cholin wird dann durch die Oxidase in Betain und $H_2O_2$ umgewandelt, das dann durch den elektrochemischen Detektor bei 500 mV konstanter Spannung nachgewiesen wird. Die Nachweisgrenze für Cholin und Acetylcholin liegt bei etwa 0,1 Picomol.

Beispiel 2

Es wurden cholesterinhaltige Proben aus menschlichem Blut in üblicher Weise für die Analyse mittels HPLC aufbereitet.

Die Proben wurden dann wie in Beispiel 1 über HPLC aufgetrennt und über eine Minisäule geleitet. Diese Minisäule war eine Glasminisäule (4 x 0,05 cm, 30 Mikroliter Volumen), die mit CNBr aktivierte Agarose enthielt, an die 5 Einheiten Cholesterinoxidase aus Nocardias erythropolis und 5 Einheiten Cholesterinesterase aus Schweinepankreas gebunden waren.

Nach Trennung des Cholesterins und der Ester auf der HPLC-Säule erfolgte die Hydrolyse der Ester und die Oxidation des Cholesterins in der Minisäule; das entstehende $H_2O_2$ wurde dann elektrochemisch nachgewiesen. Zur Eichung der Messungen wurden ein Cholesterinstandard verwendet.

Zur Durchführung der Analysen wurden jeweils 20 Mikroliter Blut verwendet. Die Nachweisgrenze war dabei 0,1 pMol Cholesterin in den Proben.

**Patentansprüche**

1. Verfahren zum Nachweis von Verbindungen, die Substrate für eine $H_2O_2$-liefernde Oxidoreduktase darstellen, unter Verwendung von HPLC und elektrochemischen Nachweismethoden, dadurch gekennzeichnet, daß man eine Probe, die eine oder mehrere zu bestimmende Verbindungen, die Substrate für eine $H_2O_2$-liefernde Oxidoreduktase darstellen, auf eine HPLC-Säule gibt und mit einem geeigneten Elutionsmittel eluiert; das Eluat dann durch eine, mit einer Trägersubstanz gepackten Minisäule leitet, an der die Oxidoreduktase und gegebenenfalls Hilfsenzyme gebunden sind, die Verbindungen in Substrate für eine $H_2O_2$-liefernde Oxidoreduktase umwandeln, wobei der pH des Eluats nicht verändert wird und anschließend in der, die Minisäule verlassende Lösung die von der Oxidoreduktase oxidierte oder reduzierte Verbindung durch elektrochemische Detektion quantitativ bestimmt.

2. Verfahren zum Nachweis von Acetylcholin und Cholin unter Verwendung von HPLC und elektrochemischen Nachweismethoden, dadurch gekennzeichnet, daß man eine Probe, die Acetylcholin und Cholin enthält, über eine HPLC-Säule auftrennt, mit einem Puffer bei pH 7 eluiert, das Eluat dann durch eine mit einer Trägersubstanz, an die immobilisierte Acetylcholinesterase und Cholinoxidase gebunden ist, gepackte Minisäule leitet und anschließend in der die Minisäule verlassenden Lösung das aus Cholin gebildete $H_2O_2$ elektrochemisch quantitativ bestimmt.

3. Verfahren nach Anspruch 1, dadurch gekennzeichnet, daß die nachzuweisenden Verbindungen Cholesterin und Cholesterinester sind und als immobilisierte Enzyme Cholesterinoxidase und Cholesterinesterase verwendet werden.

**Revendications**

1. Procédé de mise en évidence de composés qui constituent des substrats pour une oxydoréductase fournissant du $H_2O_2$, en utilisant la HPLC et des méthodes de détection électrochimiques, caractérisé en ce qu'on verse sur une colonne de HPLC un échantillon qui contient un ou plusieurs composés à déterminer, qui constituent des substrats pour une oxydoréductase fournissant du $H_2O_2$, et en ce qu'on élue avec un éluant approprié; en ce qu'on envoie ensuite l'éluat à travers une mini-colonne remplie d'une substance de support à laquelle sont liées l'oxydoréductase et

le cas échéant dès enzymes auxiliaires qui transforment les composés en substrats pour une oxydoréductase fournissant du $H_2O_2$, le pH de l'éluat n'étant pas modifié, puis en ce qu'on détermine quantitativement par détection électrochimique, dans la solution sortant de la mini-colonne, le composé oxydé ou réduit par l'oxydoréductase.

2. Procédé de mise en évidence de l'acéthylcholine et de la choline en utilisant la HPLC et des méthodes de détection électrochimiques, caractérisé en ce qu'on sépare l'échantillon qui contient de l'acétylcholine et de la choline sur une colonne d'HPLC, en ce qu'on élue avec un tampon à pH 7, en ce qu'on envoie ensuite l'éluat à travers une mini-colonne remplie d'une substance de support sur laquelle sont liées de l'acétylcholine-estérase et de la choline-oxydase immobilisées, puis en ce qu'on détermine quantitativement par voie électrochimique, dans la solution sortant de la mini-colonne, le $H_2O_2$ formé à partir de la choline.

3. Procédé suivant la revendication 1, caractérisé en ce que les composés à détecter sont le cholestérol et les esters de cholestérol et en ce qu'on utilise comme enzyme immobilisée la cholestéroloxydase et la cholestérolestérase.

**Claims**

1. Process for the detection of compounds which represent substrates for an $H_2O_2$-producing oxidoreductase with the use of HPLC and electrochemical detection methods, characterised in that one applies a sample which contains one or more compounds to be determined, which represent substrates for an $H_2O_2$-producing oxidoreductase, to an HPLC column and elutes with a suitable elution agent; then passes the eluate through a minicolumn packed with a carrier substance to which are bound the oxidoreductase and possibly adjuvant enzymes, converts the compounds into substrates for an $H_2O_2$-producing oxidoreductase, whereby the pH of the eluate is not changed, and subsequently quantitatively determines in the solution leaving the minicolumn, by electrochemical detection, the compound oxidised or reduced by the enzyme.

2. Process for the detection of acetylcholine and choline with the use of HPLC and electrochemical detection methods, characterised in that one separates a sample which contains acetylcholine and choline over an HPLC column, elutes with a buffer at pH 7, then passes the eluate through a minicolumn packed with a carrier substance to which immobilised acetylcholine esterase and choline oxidase are bound and subsequently quantitatively determines electrochemically in the solution leaving the minicolumn the $H_2O_2$ formed from choline.

3. Process according to claim 1, characterised in that the compounds to be detected are cholesterol and cholesterol esters and cholesterol oxidase and cholesterol esterase are used as immobilised enzymes.